Europäisches Patentamt

European Patent Office

Office européen des brevets

(11) Publication number: 0 165 718
A2

# (12) EUROPEAN PATENT APPLICATION

(21) Application number: 85303592.1

(22) Date of filing: 21.05.85

(51) Int. Cl.⁴: **A 61 B 1/06**
**A 61 B 1/04**

(30) Priority: 22.05.84 GB 8413058

(43) Date of publication of application:
27.12.85 Bulletin 85/52

(84) Designated Contracting States:
AT BE CH DE FR GB IT LI LU NL SE

(71) Applicant: Pilkington Medical Systems Limited (formely Minvade Limited)
Bleasdale Court 2 South Avenue Clydebank Business Park
Clydebank G81 2LE Scotland(GB)

(72) Inventor: Evans, John Yorath Gwynne
Rushmoor Cottage
Tilford Farnham Surrey(GB)

(72) Inventor: Grantham, Anthony Charles
24 Kingsmead
Smallfield Surrey(GB)

(74) Representative: Meddle, Alan Leonard et al,
FORRESTER & BOEHMERT Widenmayerstrasse 4/I
D-8000 München 22(DE)

(54) Endoscopes.

(57) An endoscope has an insertion tube (2) constructed from a plurality of disks (15) strung together on steering cables (6) and received in a flexible plastics sheath. Each disk is formed with a plurality of service apertures defining a plurality of service passages of equal diameter within the chain of disks (15). The cable apertures (16) in the disks (15) are skewed so that each cable (6) follows a helical path along the length of the insertion tube (2).

Fig.1.

EP 0 165 718 A2

"Endoscopes"

THIS INVENTION relates to improvements in or relating to endoscopes.

A known endoscope comprises a flexible insertion tube for insertion into a cavity to be investigated, such as a body cavity of a patient in the case of a medical endoscope, such flexible tube having a leading or distal end section which is capable of angulation by means of steering or angulation mechanism contained in a proximal control unit attached to a trailing or proximal end of the flexible insertion tube. The angulation section of the insertion tube can be flexed by means of steering wires connected to a steering block at the distal tip of the angulation section and extending through the insertion tube to the angulation mechanism in a proximal control unit. The angulation mechanism includes a brake to enable the mechanism to be retained in an adjusted position with the angulation section set in a desired position.

A viewing system of the endoscope comprises a first non-coherent lighting bundle of optical fibres for conducting light from a light source in a services console through the proximal control unit and the insertion tube to a window or windows in the distal tip of the angulation section. A lens provided in the distal tip of the angulation section collects reflected light and focuses it onto one end of a coherent image bundle of optical fibres which extends through the insertion tube to an eye piece provided on the proximal control unit for viewing the image transmitted by the image bundle.

The services console also provides supplies of pumped water and compressed air which are supplied to respective outlets in the distal tip of the angulation section via a control valve arrangement in the proximal control unit and respective conduit tubes extending through the insertion tube.

In the known endoscope, the flexible insertion tube is constructed from a chain of annular metal elements or vertebrae, adjacent ones of which are pivotally connected to one another at diametrically opposite positions. Each vertebra is provided with apertured lugs which serve as guides for the steering wires. The chain of metal vertebrae is covered with a flexible sheath of plastics material or the like and defines a single central internal services passage through which extend the lighting and image optical fibre bundles, the air and water supply tubes and possibly a biopsy tube for guiding to a biopsy outlet at the distal tip a biopsy instrument insertable into the biopsy tube at the proximal control unit.

The construction of the insertion tube of the know endoscope has the disadvantage that all of the services of the endoscope pass through the single central channel formed by the chain of vertebrae and this means that the various items are prone to undergo relative movement and rub or press against one another and the vertebrae when the insertion tube is flexed or coiled, as it frequently is. Moreover, this know construction means that the delicate internal service components, such as the optical fibre bundles, must be installed at the early stages of assembly and are subjected to potential damage during the assembly, adjustment and testing of the mechanical components, such as the angulation mechanism and steering wires.

It is one object of the present invention to provide an improved insertion tube construction tube for an endoscope and, to this end, the present invention provides an endoscope having an insertion tube constructed from a plurality of disks so constructed and connected to as to form an articulated chain enclosed in an outer sheath of flexible material, the disks being formed with a first plurality of guide apertures for receiving retaining wires by which the chain of disks is held together and a second plurality of service apertures aligned with corresponding apertures in the other disks to form a plurality of separate service passages extending through the insertion tube and each for receiving and confining a respective service component.

Such a construction of the insertion tube not only permits the loading of delicate internal components after the rest of the endoscope has been assembled, adjusted and mechanically tested, but also facilitates the later addition or replacement of delicate service components in the insertion tube without interference with the other service components.

Preferably, at least some of the service passages have a common standard transverse dimension so as to enable the passages to serve interchangeable to accomodate different service components or accessories and to provide a modular system in which tested and proven sub-assemblies, such as a coherent bundle with its prefocussed optical system, can be inserted or interchanged as required to suit particular circumstances.

According to the invention, in another aspect thereof, there is provided an endoscope having an insertion tube constructed from a chain of disks enclosed within a flexible outer sheath, in which each disk has a service aperture defining with corresponding aligned apertures of the other disks a service passage extending through the chain of disks and in which the disks are articulated together in a manner which minimizes relative movement between the disks transversely of the service passage upon bending of the insertion tube.

In one embodiment of the invention, the disks of the insertion tube are held together by retaining wires, each of which passes through a guide aperture near the periphery of each disk and spirals about the longitudinal axis of the insertion tube so as to execute one or more substantially complete turns in the course of the length of the insertion tube. The spiral configuration of the retaining wires may be acheived by inclining each guide aperture in a disk by a predetermined amount relative to the axis of the disk.

The retaining wires may be constituted by steering cables for steering an angulation section at the distal end of the insertion tube.

In another embodiment of the invention, the steering cables for steering an angulation section of the insertion tube travel centrally through the disks of the insertion tube from the proximal end of the insertion tube and are guided outwardly to positions near the periphery of the angulation section at the start of the angulation section by a cable-diverting device positioned between the insertion tube proper and the angulation section.

According to a further aspect of the invention, there is provided an endoscope having an insertion tube constructing from a chain of tube elements which are pivotally interconnected and retained by retaining wires, in which alternate tube elements have a pair of diametrically opposed apertured projections for reception in corresponding recesses of an adjacent tube element to constitute a pivotal connection between the two tube elements, each apertured projection also serving as a guide through which a corresponding retaining wire extends.

The tube elements of an insertion tube of an endoscope embodying the invention may be in the form of an annular element with a single central service passage or maybe in the form of a disk provided with a plurality of separate service passages.

Another operating problem of the known endoscope described in the introduction arises from the fact that stressing of the control cables when the insertion tube is bent (and particularly when coiled) can lead to stretching of the cables and consequent play in the steering mechanism for steering the angulation section of the insertion tube. Further, the capstan arrangement used to tension the steering cables in the know endoscope construction is not able to accomodate the differential movement which occurs between the steering cables which are on the inside and outside of the curve during bending of the angulation section. This can result in kinking of the untensioned cable as it is paid out in equal measure to the winding on of the tensioned cable on the inside of the curve.

It is a further object of the present invention to provide an endoscope with an improved angulation mechanism and, to this end, there is provided an endoscope comprising a proximal control unit containing an angulation mechanism, an insertion tube having an angulation section at its distal end and control cables extending from the angulation mechanism in the proximal control unit to the angulation section for angling the angulation section relative to the longitudinal axis of the insertion tube in response to operation of the angulation mechanism, in which endoscope each steering cable comprises an inner steering wire and an outer steering sheath, the proximal end of each steering wire is attached to a movable actuating member of the angulation mechanism by which the steering wire can be tensioned, the distal end of the steering wire is attached to a distal tip of the angulation section, the distal end of the steering sheath is attached to the proximal end of the angulation section, and the proximal end of the steering sheath is attached to a displaceable compensating block which is resiliently mounted for movement relative to the actuating members of the angulation mechanism, the spring-loading of the compensating block being such that the block remains substantially fixed during actuation of a single steering cable to impart motion to the angulation section but enables the compensating block to move relative to the actuating members as a result of tensioning of the steering cables upon coiling of the insertion tube.

According to yet another aspect of the invention, there is provided an endoscope having aproximal control unit provided with an angulation mechanism connected by steering cables to an angulation section at the distal end of an insertion tube, in which each of a pair of steering cables is mounted on a respective one of a pair of movable actuating members adapted to be moved by a common drive member, and the drive member is coupled to the actuating members so as to drive the actuating members together in either of two drive directions, the arrangement being such that in each drive direction a respective one of the actuating members is enabled to lag behind the other acutating member by up to a predetermined displacement.

In one form of the invention, each of the actuating members comprises a pulley wheel mounted on a drive shaft provided with a dutch pin which engages a notch in the internal periphery of each drive pulley, the notch having an angular extent about the axis of the drive shaft which is greater than the angular extent of the dutch pin.

According to yet a further aspect of the invention, there is provided an endoscope having a proximal unit provided with an angulation mechanism for actuating by steering cables an angulation section provided at a distal end of an insertion tube of the endoscope, which angulation mechanism comprises first and second drive shafts for driving actuating members disposed within the housing of the proximal unit and serving to actuate the steering cables, first and second coaxial shafts coupled to the actuating members within the proximal unit housing and extending out through such housing and means mounted on the drive shafts externally of the proximal control unit for rotating the shafts independently of one another and for retaining each shaft in a selected angular position.

According to still another aspect of the invention, there is provided an endoscope having a proximal control unit comprising a housing connected to a proximal end of a flexible insertion tube by a coupling device enabling limited rotation of the insertion tube about its axis relative to the proximal control unit housing In order that the invention may be readily understood, embodiments thereof will now be described, by way of example, with reference to the accompanying drawings, in which:

FIGURE I is an overall fragmentary side view of an endoscope embodying the present invention, with parts broken away and parts enlarged to show detail of the construction;

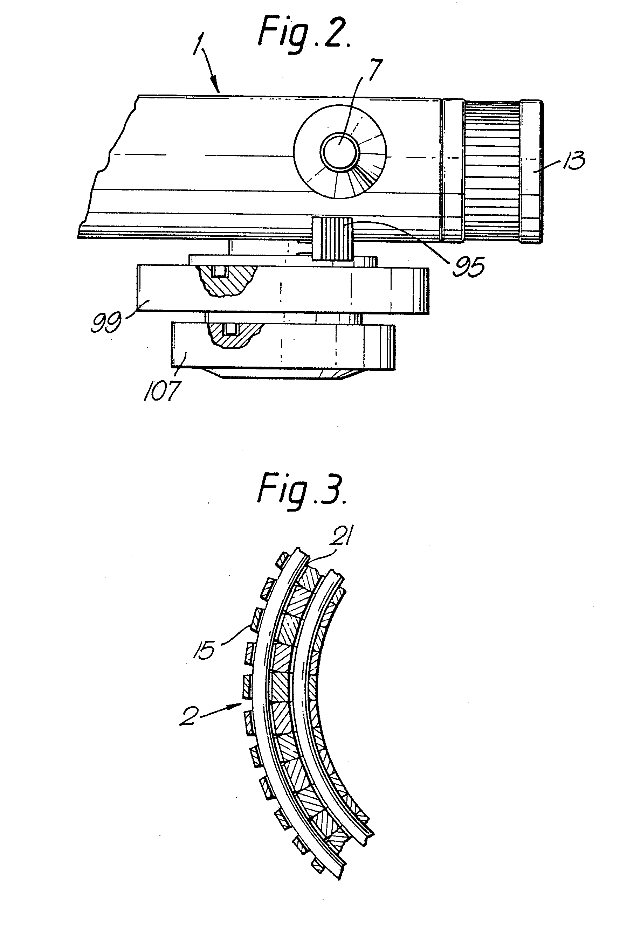

FIGURE 2 is a partial plan view of a proximal unit of the Figure I endoscope;

FIGURE 3 is an axial cross-sectional view of part of the insertion tube of the Figure I endoscope;

FIGURE 4 is an axial cross-sectional view of a distal part of the angulation section of the Figure I endoscope;

FIGURES 5A and 5B are respectively a plan view and a side view of a disk used in constructing the insertion tube of the Figure I endoscope;

FIGURE 6 shows in more detail the construction of the angulation section of the Figure I endoscope;

FIGURE 7A shows an alternative arrangement of steering cables in another embodiment of an endoscope according to the invention;

FIGURE 7B is a view on arrow X in Figure 7A;

FIGURES 8A and 8B are respectively an end view and a section on the line A - A of Figure 8A;

FIGURE 9 illustrates another form of insertion tube construction for another embodiment of an endoscope according to the present invention;

FIGURES 10A to 10D show respectively a tube element at one end of the insertion tube, a first kind of intermediate tube element, a second kind of intermediate tube element and a tube element at the other end of the insertion tube;

FIGURE 11 illustrates a spring-loaded anchor block of the angulation mechanism provided in the proximal control unit of the Figure 1 endoscope;

FIGURE 12 illustrates the drive for cable actuating members of the angulation mechanism of the Figure 1 endoscope;

FIGURE 13 illustrates the manual controls, braking and stop arrangements of the angulating mechanism of the Figure 1 endoscope; and

FIGURE 14 is an axial cross-sectional view of a polar motion mechanism of the Figure 1 endoscope.

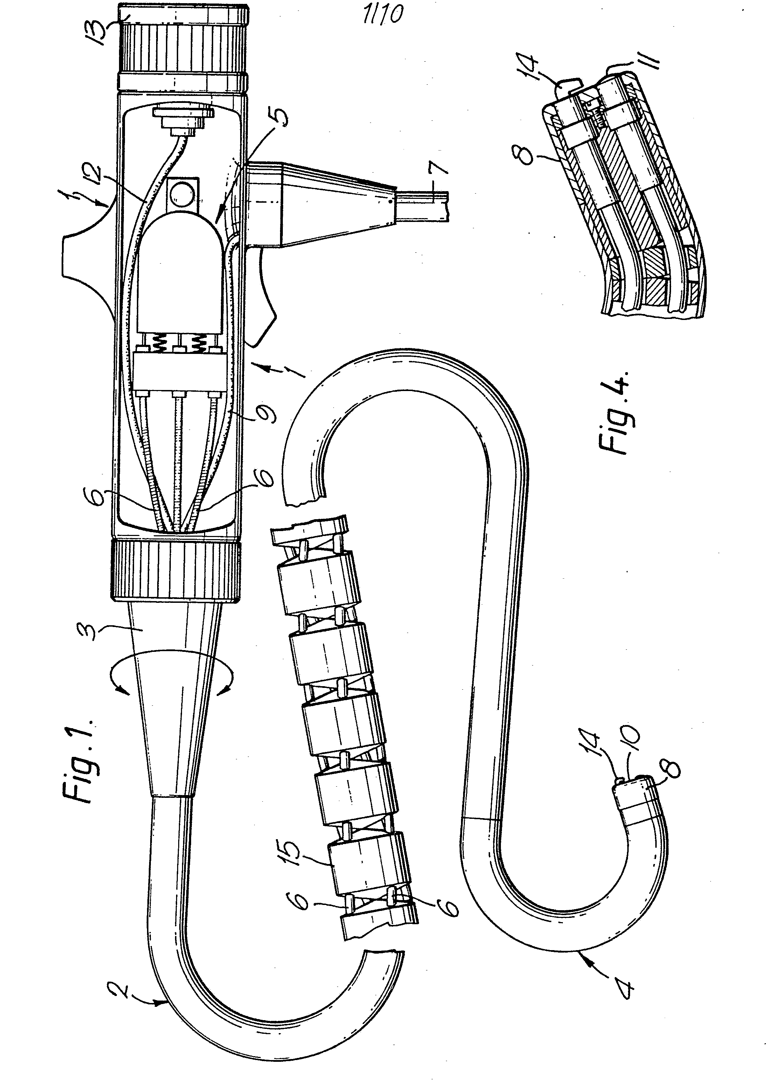

Referring firstly to Figure 1, an endoscope embodying the present invention comprises a proximal control unit 1 connected to the proximal end of an insertion tube 2 by a polar motion coupling 3 enabling limited relative movement between the housing of the proximal control unit 1 and the insertion tube 2 about the longitudinal axis of the insertion tube.

The insertion tube 2 is terminated at its distal end by an angulation section 4 which can be angled relative to the rest of the flexible insertion tube 2 by means of an angulation mechanism 5 transmitting movement to the angulation section 4 via four steering cables 6, of which only two are visible in Figure 1. A service tube 7 connects the proximal control unit to a services console (not shown) which contains a light source, an air pump and a water pump. Light is fed to the distal tip 8 of the angulation section of the insertion tube by means of a non-coherent bundle 9 of optical fibres which passes from the service console through the service tube 7 and the proximal control unit into the insertion tube 2 and thence to the distal tip 8 where light from the end of the bundle emerges through a window provided in the end face 10 of the distal tip. Reflected light is received by a lens system 11 mounted in the distal tip 8 and transmitted via a coherent bundle 12 of optical fibres to an eye piece 13 for viewing the transmitted image. Air and water from the service console are supplied via pipes extending through the service tube 7 to a valve (not shown) which controls the delivery of the air and water through respective supply pipes to a jet 14 projecting from the end face 10 of the distal tip 8 and angled towards the lens system 11.

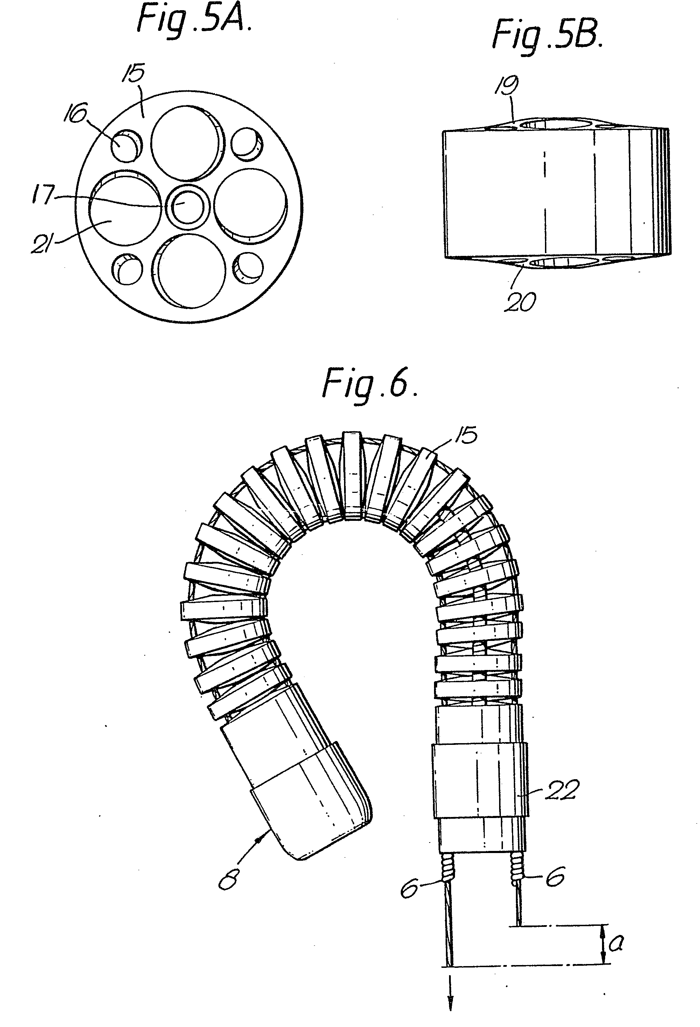

The insertion tube 2 is constructed from a chain of tube elements or vertebrae 15 which are strung together on the steering cables 6 and received in a flexible sheath 2A of plastics or the like. As shown more particlarly in 5A, each of the vertebrae 15 is in the form of a disk, made of plastics, for example, provided with four guide apertures 16 positioned near the periphery of the disk and each for receiving a respective steering cable 6. An aperture 17 at the centre of the disk 15 accomodates a central compensation cable 18 (Figure 1). As shown in Figures 1 and 5B, limited relative pivoting movement is permitted between adjacent disks 15 by virtue of their frusto-conical end faces 19 and 20.

Each disk 15 is formed with four large service apertures of equal diameter, whereby four service passages of equal diameter are defined within the chain of disks 15 constituting the insertion tube. The angulation section of the insertion tube is constructed in a similar fashion to the rest of the insertion tube but using disks 15 of smaller axial dimension as shown in Figure 6, which shows the angulation section of the insertion tube without its external flexible sheath 2A. Still referrring to Figure 6 it will be seen that the angulation section includes distal tip 8 and an anchor block 22 disposed between the angulation section and the rest of the insertion tube.

As can be most clearly seen from Figures 1 and 5A, the cable apertures 16 in the disks 15 are skewed so that the two end faces 19 and 20 of the disk are effectively rotated relative to each other by about $4.5^{\circ}$. As a result, each cable 6 follows a helical path along the length of the insertion tube 2 so that it executes a complete revolution about the longitudinal axis of the insertion tube in the length of the insertion tube. On the other hand, the disks employed in the angulation section of the insertion tube have axial bores so that the steering cables 6 run parallel to the axis in this section, as shown in Figure 6.

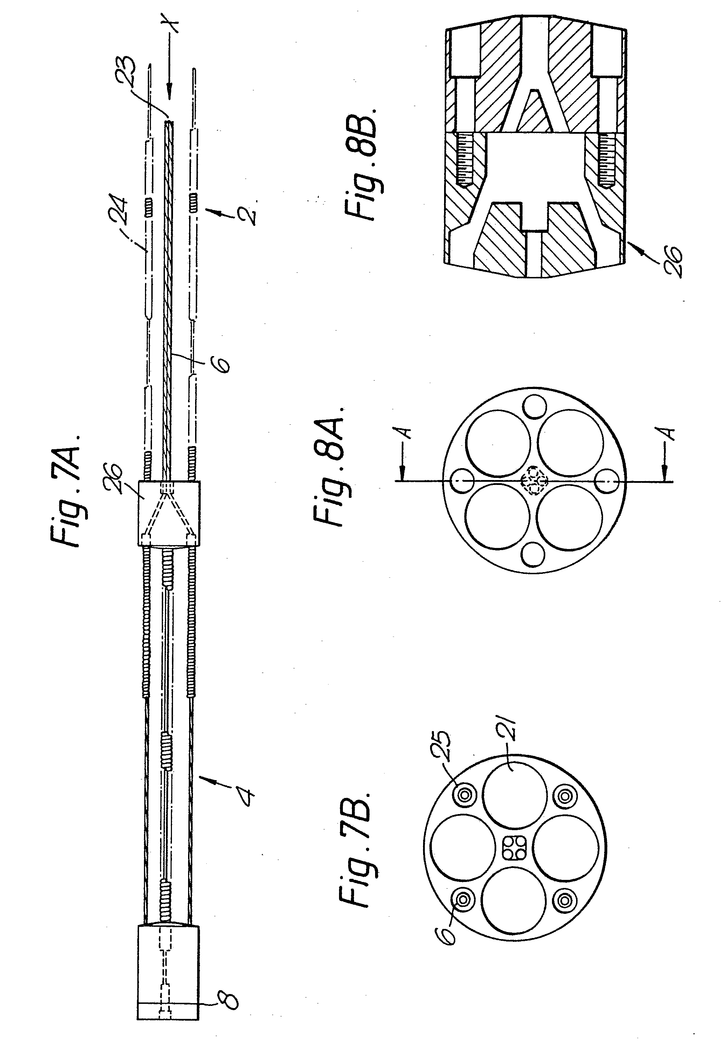

In another embodiment of the invention illustrated in Figures 7A, 7B, 8A and 8B, the steering cables 6 extend axially through the insertion tube 2 at locations close to the longitudinal axis 23 as shown in Figure 7A. In this case, separate retaining cables 24 are threaded axially through connection apertures 25 provided near the periphery of the disks 15. The transition from the insertion tube proper to the angulation section 4 is effected by means of a diverter block 26 which diverts the course of the wires of steering cables 6 from a position close to the axis of the insertion tube to a position close to the periphery of the angulation section, in which region each cable 6 passes through a cable aperture disposed near the periphery of each of the disks 15 in the angulation section 4.

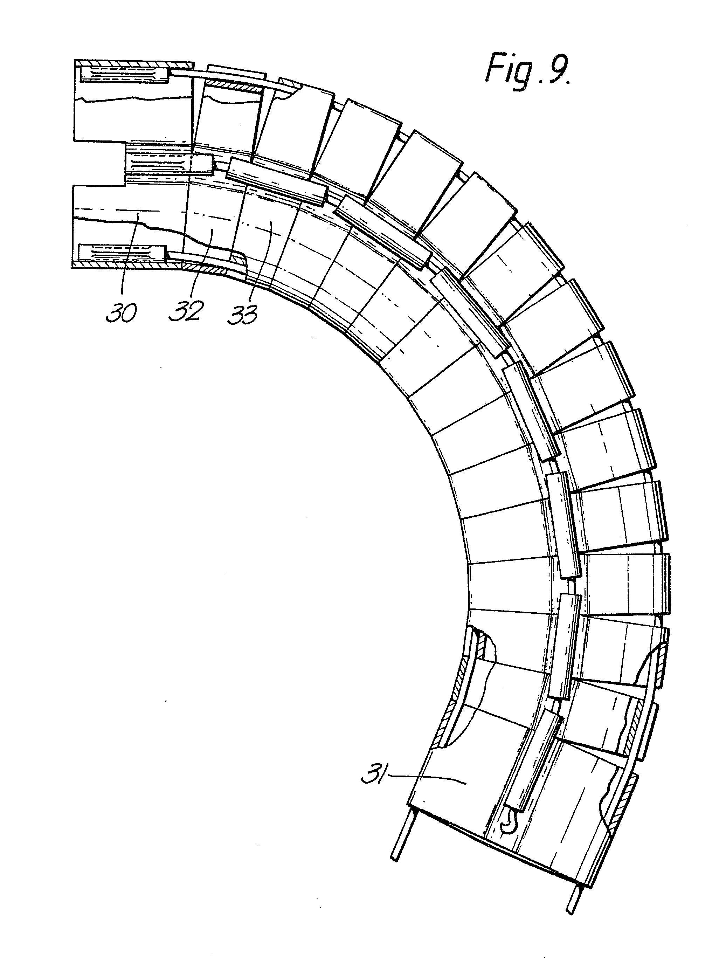

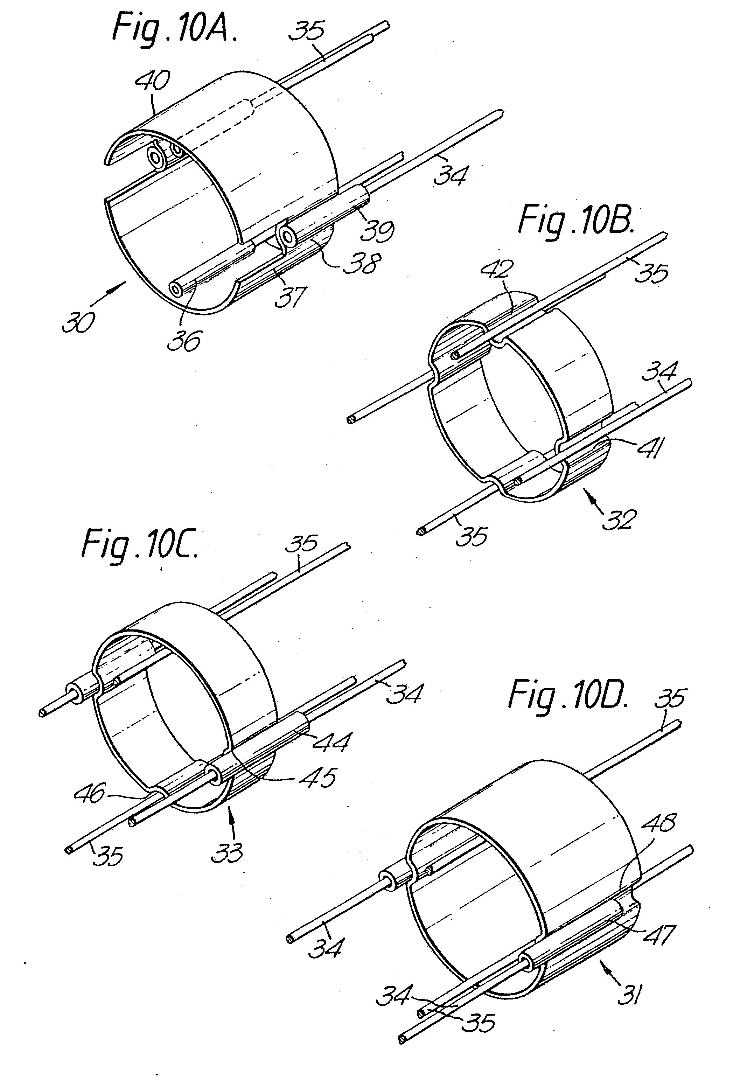

Figures 9 and 10A to 10D illustrate another way in which the insertion tube of an endoscope may be constructed in accordance with the present invention. In the case of an endoscope, such as a nephroscope, in which there is only requirement for movement of the angulation section in a single plane as can be seen from Figure 9, which shows an angulation section of the insertion tube of the endoscope the insertion tube is constructed from four different types of tube element, namely a distal end tube element 30, a proximal end tube element 31 a female tube element 32 and a male tube element 33. The female and male elements 32 and 33 are used alternately to construct the tube portion between the end units 30 and 31, the latter serving as male tube elements and engaging adjacent female tube elements 32. The tube elements 30 to 33 are strung together on retaining cables 34 and the inner wires 35 of a pair of steering cables 36 are threaded through the chain of tube elements to emerge from the proximal end tube element 31 from whence they pass to the angulation mechanism (not shown) of the endoscope.

Figures 10 to 10D show in detail the construction of the four different kinds of tube element 30 to 33, each of which consists essentially of a short cylindrical metal member which accomodates the cables 34 and 35 at its periphery and defines with the other tube elements a single central service passage through the insertion tube.

The distal end tube element 30 shown in Figure 10A comprises a substantially cylindrical metal ring 37 having a pair of diametrically opposite recesses 38 in which are retained cable terminations 39 receiving the ends of the retaining cables 34. A pair of cable terminations 40 on the distal ends of the steering cables 35 are secured to the inside periphery of the element 30 at diametrically opposed locations displaced 90° around the circumference from the recesses 38.

Figure 10B shows a female tube element 32 which is provided with diametrically opposed guide recesses 41 for the passage of the retaining cables 34 and which also serve for coupling to adjacent male tube elements as hereinafter described. One of the steering cables 35 is accomodated in a recess 42 midway betweent the recesses 41 and the other steering cable 35 passes freely through the tube elements 32 adjacent the internal surface of the tube 32 at a position diametrically opposite the recess 42.

The male tube element 33 shown in Figure 10C has diametrically opposed hollow projections or pegs 44 seated in recesses 45 formed in the tube 33 and projecting axially on both sides of the element 33. The retaining cables 34 pass through these hollow pegs while the steering cables 35 which passes through the recess 42 of an adjacent female element 32. The steering cable 35 which passes freely through an adjacent female tube element 32 is accomodated externally of the male tube element 33 in a recess 46.

The proximal end tube element 31 shown in Figure 10D has a pair of cable anchors 47 disposed in diametrically opposed recesses 48 of the element 31 and serving to terminate the proximal ends of the retaining cables. The cable anchors 47 are cylindrical and project axially from the element 31 on one side for engagement in recesses 41 of the adjacent female element 32.

Figure 9 shows the assembled string of tube elements and shows that the ends of the tubes are slightly conical in side elevation to permit relative pivoting movement of adjacent tube elements in the plane defined by the pair of steering cables 35. The engagement of the hollow pegs 44 and the cable anchors 38 and 47 or the male tubular members in the recesses of 41 of adjacent female elements can be clearly seen in Figure 9.

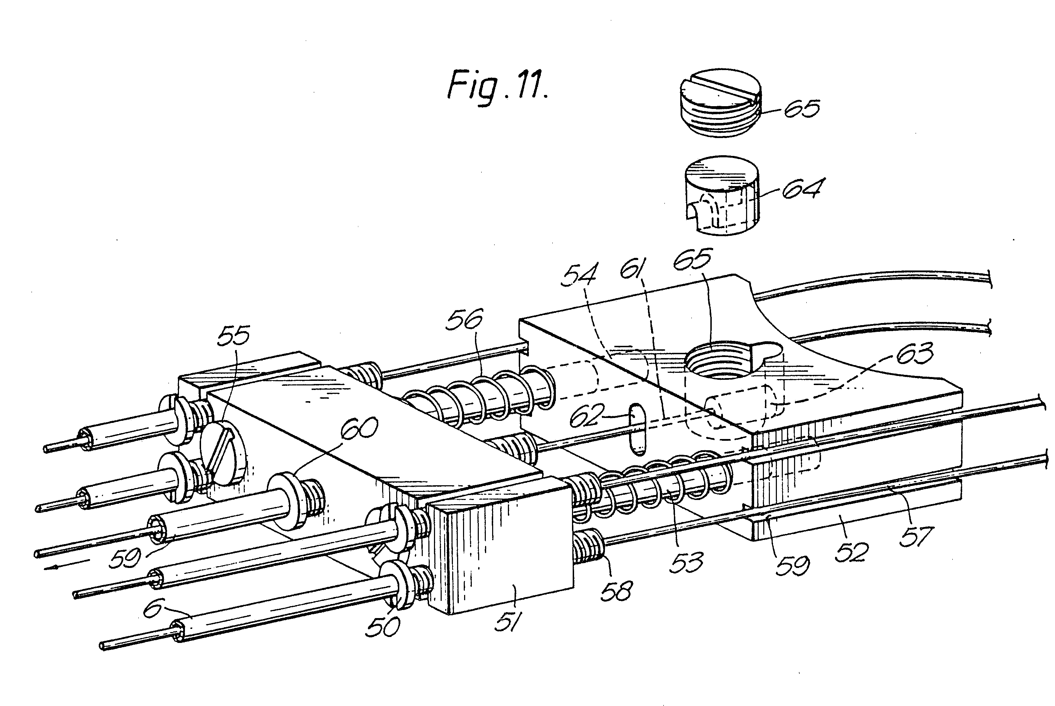

Figure 11 of the drawings shows the mounting of the proximal ends of the steering cables of the Figure 1 endoscopes. As shown in Figure 11, the outer sheaths of the steering cables are each terminated by a hollow adjusting screw 50 which is screwed into a corresponding threaded through bore formed in a spring-loaded anchor lock 51. Anchor block 51 is coupled to a main body 52 of the angulation mechanism by a pair of plungers 53 which are passed through smooth bores in the block 51 and have threaded ends 54 which screw into the main body 52. The unthreaded part of the plunger is terminated by a head 55 against which the block 51 is normally pressed by a compression spring 56. The inner wires 57 emerge from the ends 58 of the adjusting screws 58 and are guided in grooves 59 formed in the main body 52. The steering wires 57 are attached to respective actuating elements (not shown in Figure 11) for tensioning the wires in order to flex the angulation section of the endoscope. The spring rating of the springs 56 is selected so that, when of the wires 57 is tensioned in normal operation to flex the angulation section of the endoscope, the loaded block remains substantially fixed in position pressed against the heads 55 of the plungers 53. On the other hand, when the insertion tube 2 is flexed or coiled the combined tensions induced in the steering cables causes the anchor block 51 to be displaced towards the fixed main body 52 to accomodate the relative movement between the cables and to avoid undue stressing and stretching of the cables. A central compensating cable has its outer sheath terminated by an adjusting screw 60 screwed into a corresponding threaded through ball in the anchor block 51. The inner wire 61 of the cable is received in a bore 62 in the main body 52 and has a nipple 63 attached to its end. The nipple 63 is secured in the main body 52 by a nipple anchor 64 introduced into the main body through a threaded further bore 65 transverse to the bore 62. The little anchor is retained by a lock screw 65 screwed into the bore 65 after the insertion of the nipple 64.

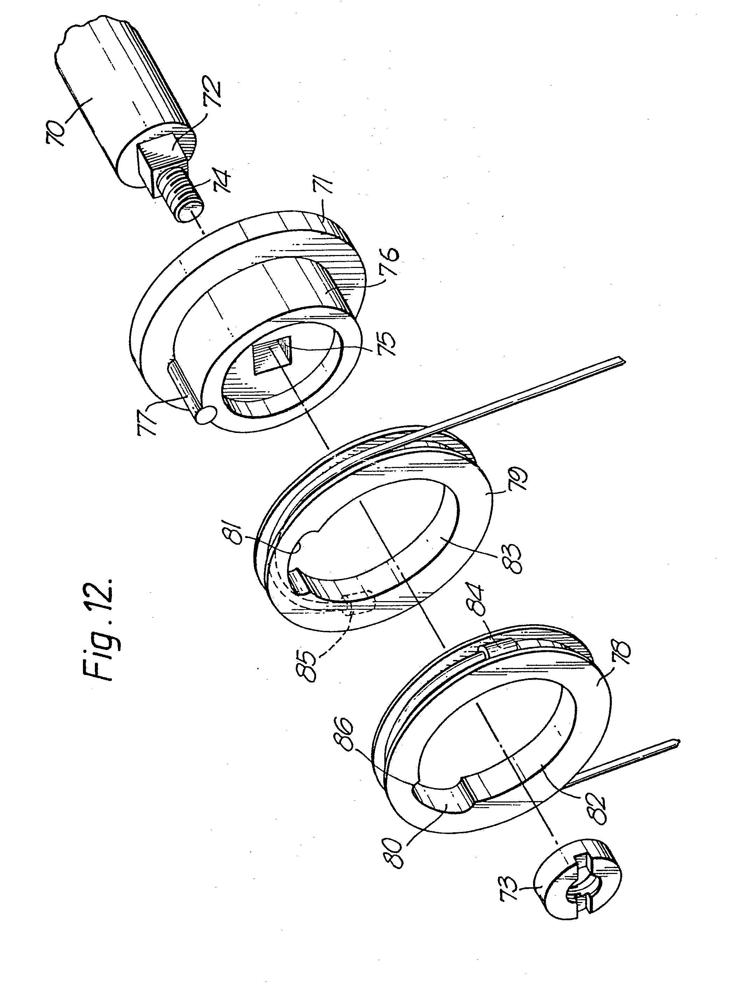

The means for actuating each of the two pairs of cables 6 is shown in Figure 12 of the drawings. The two cables 6 shown therein define a plane in which the angulation section 4 of the endoscope of Figure 1 may be flexed. By tensioning one or other of the two cables 6, the angulation section 4 of the endoscope of Figure 1 may be made to bend in a respective direction.

-12- 0165718

Whilst tensioning one cable, the other cable must be paid out. The arrangement shown in Figure 12 tensions 1 cable and pays out the other cable at the same time, whilst allowing the rate of winding in and paying out to differ within limits within the two cables in order to accomadate any differential stretching of the cables which may have taken place in use. The drive arrangement shown comprises a steering shaft 70 manually rotatable by a control knob (not shown in Figure 12) keyed to the shaft. A drive flange 71 is keyed onto a square section boss 72 projecting axially from the steering shaft and is retained by a lock nut 73 received on a threaded stud 74 projecting from the boss 72. The steering flange 71 has an axially through hole 75 of square cross-section receiving the boss 72 of the steering shaft 70. The drive flange 71 is formed with an integral cylindrical hub 76 provided with a dutch pin 77 on its circumference. First and second actuating pulleys 78 and 79 are receivable on the hub 76 so that the dutch pin 77 extends through arcuate notches 80 and 81 formed in the inner rings 82 and 83 of the pulleys. Each pulley has a grooved outer rings containing a cable anchor 84, 85 for a respective cable. As seen from the left of Figure 12, the steering cable anchored to pulley 78 extends anticlockwise around the pulley from the cable anchor and the steering cable anchored to pulley 79 extends clockwise around the pulley from the cable anchor. Upon clockwise rotation of the shaft 70, the respective steering cable is wound onto pulley 78 as a result of the dutch pin 77 engaging the leading shoulder 86 of the notch 80. At the same time, the other steering cable is paid off from the other pulley 79 as necessary. Note, however, that the notch 81 has an angular extent greater than that of the dutch pin 77 and thus permits the movement of the pulley 79 to lag behind the movement of the pulley 78 by up to a predetermined amount to compensate for passed differential stretching of the two steering cables. Similarly, when the shaft 70 is rotated in the anticlockwise direction, the movement of the pulley 78 is allowed to lag behind the movement of the pulley 79 to the degree permitted by the angular extent of the notch 80.

Figure 12 illustrates the mechanism for rotating two coaxial steering shafts for driving respective pairs of steering pulleys as in Figure 12. The mechanism illustrated in Figure 12 comprises a first solid steering shaft 70 arranged within a second hollow steering shaft 90. The two shafts 70 and 90 extend through and are rotatably mounted within a fixed assembly 91 which is non-rotatably secured in an aperture of the housing of the proximal control unit so that a flange 92 of the assembly provided a multi-start screw thread 93 is disposed outside the proximal unit housing. A braking ring 94 has a multi-start thread 95 corresponding to the thread 93 and is received on the flange 92 of the fixed assembly 91. An operating lever 95 projects from the braking rings 94 and enables the rings 94 to be rotated on the flange 92. A first friction disk 96 is non-rotatably received on a first external boss 97 of the fixed assembly 91 and carries an axially extending stop pin near its periphery for engagement in a limit groove 98 forms in a confronting surface 99 of a first steering knob 100 keyed on to the hollow steering shaft 90. A circular groove 101 in the surface 99 of the steering knob 100 accomodates a friction ring 102. A second friction disk 103 is keyed onto a second external non-rotatable boss of the fixed assembly 91 and has a stop pin 105 for engagement in a part-circular limit groove 106 in the confronting surface 107 of a second steering knob 108 keyed on to the first solid steering shaft 70. A circular groove 109 in the face 107 of the steering knob 108 accomodates a ring 110 of friction material.

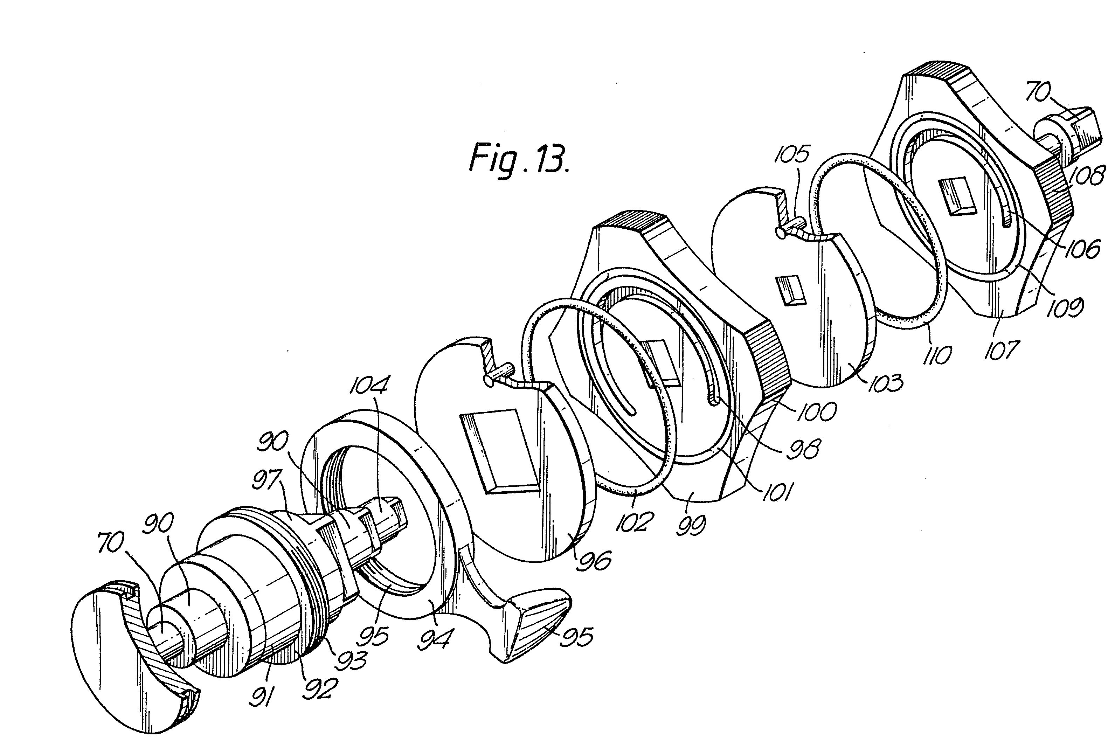

In operation of the steering mechanism described in Figure 13, clockwise rotation (as seen from the left in Figure 13) of the operating ring 94 presses each of the friction disks 96 and 103 against their respective steering wheels 100 and 108 so that the friction between the friction disk and the respective ring 102 or 110 of friction material precludes ready rotation of the steering knobs 100 and 108. Rotation of the braking ring 94 in the anticlockwise position removes the axial thrust from the friction disks 96 and 103 and allows one or other of the two steering knobs 100 and 108 to be rotated to a desired position within the limits set by the stop pins running in the limit grooves 98 and 106 of the steering knobs. A desired adjusted position of the steering knob may be retained by clockwise rotation of the braking ring 94 to apply thrust to the friction disks 96 and 103 and thereby restrain further rotation of the steering knobs.

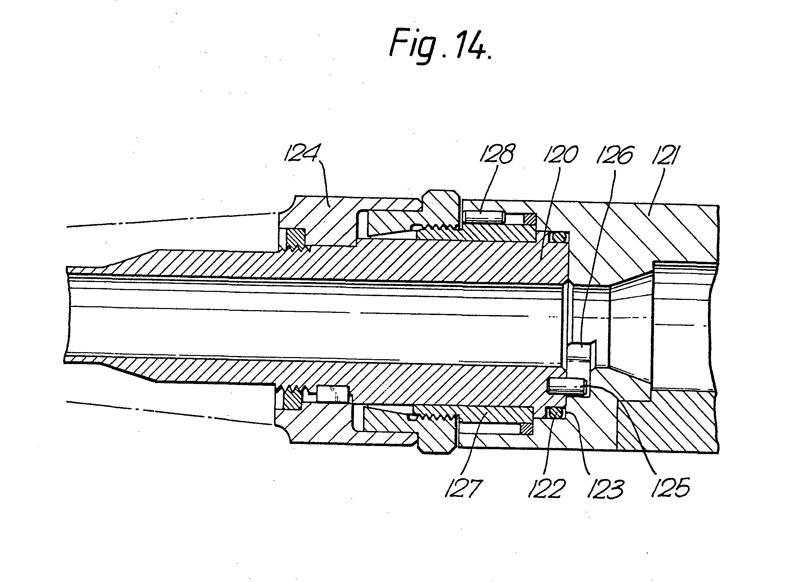

Figure 14 shows the polar motion coupling device by means of which limited relative motion between the insertion tube and the proximal control unit is enabled in the Figure 1 endoscope to enable the angulation section to be turned through a limited angle, if desired. As shown in Figure 14, the proximal end of the insertion tube is terminated by a sleeve 120 rotatable received within the housing 121 of the proximal control unit. An O ring 122 is received within a rebate 123 of the rotatable sleeve 120 by the seal between the sleeve and the housing 121. Rotation of the sleeve 120 is effected by means of an actuating ring 124 which is keyed to the rotatble sleeve 120. Rotation of the sleeve 120 by means of the acutating ring is limited by means of a stop pin 125 running in a limit groove 126 for the housing of the proximal control unit. A clamping and bearing sleeve 127 is non-rotatably mounted in the housing of the proximal control unit by means of a lock pin. The bearing sleeve has an externally threaded extension on which is mounted an internally threaded braking ring which is rotatable relative to the actuating ring to clamp the sleeve 120 in a desired positon set by the actuating ring.

It should, of course, be appreciated that the invention is applicable to endoscopes using other picture transmission arrangements, for example endoscopes using charge coupled devices (CCDS) for picture transmission as an alternative to coherent fibre bundles. Also, the services provided could include the provision of, for example, carbon dioxide as well as air and passages might be used for diathermy, laser surgery, sonic probes or other techniques.

CLAIMS

1.    An endoscope having an insertion tube constructed from a plurality of disks so constructed and connected to as to form an articulated chain enclosed in an outer sheath of flexible material, the disks being formed with a first plurality of guide apertures for receiving retaining wires by which the chain of disks is held together and a second plurality of service apertures aligned with corresponding apertures in the other disks to form a plurality of separate service passages extending through the insertion tube and each for receiving and confining a respective service component.

2.    An endoscope according to Claim 1, wherein at least some of the service passages have a common standard transverse dimension so as to enable the passages to serve interchangeable to accomodate different service components or accessories and to provide a modular system in which tested and proven sub-assemblies, such as a coherent bundle with its prefocussed optical system, can be inserted or interchanged as required to suit particular circumstances.

3.    An endoscope having an insertion tube constructed from a chain of disks enclosed within a flexible outer sheath, in which each disk has a service aperture defining with corresponding aligned apertures of the other disks a service passage extending through the chain of disks and in which the disks are articulated together in a manner which minimizes relative movement between the disks transversely of the service passage upon bending of the insertion tube.

4.    An endoscope according to Claim 3, wherein the disks of the insertion tube are held together by retaining wires, each of which passes through a guide aperture near the periphery of each disk and spirals about the longitudinal axis of the insertion tube so as to execute one or more substantially complete turns in the course of the length of the insertion tube.

5.    An endoscope according to Claim 4, wherein the the spiral configuration of the retaining wires is achieved by inclining each guide aperture in a disk by a predetermined amount relative to the axis of the disk.

6. An endoscope according to claim 4 or 5, wherein the retaining wires may be constituted by steering cables for steering an angulation section at the distal end of the insertion tube.

7. An endoscope according to Claim 3, wherein steering cables for steering an angulation section of the insertion tube travel centrally through the disks of the insertion tube from the proximal end of the insertion tube and are guided outwardly to positions near the periphery of the angulation section at the start of the angulation section by a cable-diverting device positioned between the insertion tube proper and the angulation section.

8. An endoscope having an insertion tube constructing from a chain of tube elements which are pivotally interconnected and retained by retaining wires, in which alternate tube elements have a pair of diametrically opposed apertured projections for reception in corresponding recesses of an adjacent tube element to constitute a pivotal connection between the two tube elements, each apertured projection also serving as a guide through which a corresponding retaining wire extends.

9. An endoscope comprising a proximal control unit containing an angulation mechanism, an insertion tube having an angulation section at its distal end and control cables extending from the angulation mechanism in the proximal control unit to the angulation section for angling the angulation section relative to the longitudinal axis of the insertion tube in response to operation of the angulation mechanism, in which endoscope each steering cable comprises an inner steering wire and an outer steering sheath, the proximal end of each steering wire is attached to a movable actuating member of the angulation mechanism by which the steering wire can be tensioned, the distal end of the steering wire is attached to a distal tip of the angulation section, the distal end of the steering sheath is attached to the proximal end of the angulation section, and the proximal end of the steering sheath is attached to a displaceable compensating block which is resiliently mounted for movement relative to the actuating members of the angulation mechanism, the spring-loading of the compensating block being such that the block remains substantially fixed during actuation of a single steering cable to impart motion to the angulation section but enables the compensating block to move relative to the actuating members as a result of tensioning of the steering cables upon coiling of the insertion tube.

10.    An endoscope having aproximal control unit provided with an angulation mechanism connected by steering cables to an angulation section at the distal end of an insertion tube, in which each of a pair of steering cables is mounted on a respective one of a pair of movable actuating members adapted to be moved by a common drive member, and the drive member is coupled to the actuating members so as to drive the actuating members together in either of two drive directions, the arrangement being such that in each drive direction a respective one of the actuating members is enabled to lag behind the other acutating member by up to a predetermined displacement.

11.    An endoscope according to Claim 10, wherein each of the actuating members comprises a pulley wheel mounted on a drive shaft provided with a dutch pin which engages a notch in the internal periphery of each drive pulley, the notch having an angular extent about the axis of the drive shaft which is greater than the angular extent of the dutch pin.

12.    An endoscope having a proximal unit provided with an angulation mechanism for actuating by steering cables an angulation section provided at a distal end of an insertion tube of the endoscope, which angulation mechanism comprises first and second drive shafts for driving actuating members disposed within the housing of the proximal unit and serving to actuate the steering cables, first and second coaxial shafts coupled to the actuating members within the proximal unit housing and extending out through such housing and means mounted on the drive shafts externally of the proximal control unit for rotating the shafts independently of one another and for retaining each shaft in a selected angular position.

0165718

1110

Fig.1.

Fig.4.

**Fig.2.**

**Fig.3.**

## Fig.5A.

15
16
17
21

## Fig.5B.

19
20

## Fig.6.

15
8
22
6
6
a

Fig. 7A.

Fig. 7B.

Fig. 8A.

Fig. 8B.

Fig.9.

Fig. 10A.

Fig. 10B.

Fig. 10C.

Fig. 10D.

Fig.11.

Fig. 12.

Fig.13.

0165718

## Fig.14.